# FASCICULE DE BREVET EUROPEEN

(11) **EP 2 334 739 B1**
(45) Date de publication et mention de la délivrance du brevet: **06.11.2013**
(21) Numéro de dépôt: 09747883.8
(22) Date de dépôt: 15.09.2009
(51) Int. Cl.: C09B 23/00, C09B 69/00, C09B 57/04, C09K 11/06, C08J 5/18

(54) **NOUVEAUX CHROMOPHORES, LEUR PROCEDE DE PREPARATION ET LEUR UTILISATION**
NEUE CHROMOPHORE, HERSTELLUNGSVERFAHREN DAFÜR UND IHRE VERWENDUNG
NOVEL CHROMOPHORES, METHOD FOR THE PREPARATION THEREOF, AND USE OF SAME

(30) Priorité: 15.09.2008 FR 0856179
(43) Date de publication de la demande: 22.06.2011
(73) Titulaire: Centre National de la Recherche Scientifique - CNRS, 75794 Paris Cedex 16 (FR)
(72) Inventeur: DAUTEL, Olivier, F-34070 Montpellier (FR); MOREAU, Joël, F-34000 Montpellier (FR); LERE-PORTE, Jean-Pierre, F-34090 Montpellier (FR)
(74) Mandataire: Cabinet Plasseraud
(86) Numéro de dépôt international: PCT/FR2009/051735
(87) Numéro de publication internationale: WO 2010/029274

(56) Documents cités:
- WO-A-2007/120788
- OLIVIER J. DAUTEL, GUILLAUME WANTZ, ROBERT ALMAIRAC, DAVID FLOT, LIONEL HIRSCH, JEAN-PIERRE LERE-PORTE ET. AL.: "Nanostructuration of Phenylenevinylenediimide-Bridged Silsesquioxane: From Electroluminescent Molecular J-Aggregates to Photoresponsive Polymeric H-Aggregates" JOURNAL OF THE AMERICAN CHEMICAL SOCIETY, vol. 128, no. 14, 21 mars 2006 (2006-03-21), pages 4892-4901, XP002538565 cité dans la demande
- WANTZ G ET AL: "Layered organic film growth by substrate temperature tuning for efficiency-enhanced OLEDs" 1 février 2006 (2006-02-01), ORGANIC ELECTRONICS, ELSEVIER, AMSTERDAM, NL, PAGE(S) 38 - 44 , XP024972750 ISSN: 1566-1199 vol. 7, [extrait le 2006-02-01] cité dans la demande figure 1
- OLIVIER J. DAUTEL, GUILLAUME WANTZ, DAVID FLOT, JEAN-PIERRE LERE-PORTE, JOËL J. E. MOREAU ET. AL.: "Confined photoactive substructures on a chiral scaffold: the design of an electroluminescent polyimide as material for PLED**" JOURNAL OF MATERIALS CHEMISTRY, vol. 15, 2 septembre 2005 (2005-09-02), pages 4446-4452, XP002538566 cité dans la demande
- RONALD GRIGG, STEPHEN BROWN, VISUVANATHAR SRIDHARAN, MICHAEL D UTTLEY: "Intermolecular Heck-Diels-Alder cascade processes of alkylallenes" TETRAHEDRON LETTERS, vol. 39, no. 20, 14 mai 1998 (1998-05-14), pages 3247-3250, XP002538567
- WANTZ G ET AL: "Temperature-dependent electroluminescence spectra of organic light emitting diodes based on thermally evaporated bis-imido-phenylene vinylene derivative" 17 avril 2007 (2007-04-17), APPLIED PHYSICS LETTERS, AIP, AMERICAN INSTITUTE OF PHYSICS, MELVILLE, NY, US, PAGE(S) 162104 - 162104 , XP012094211 ISSN: 0003-6951 pages 162104-1

## Description

La présente invention porte sur de nouveaux chromophores, sur leur procédé de synthèse et sur leur utilisation.

Dans la présente demande de brevet, on entend par « chromophore », une molécule de type pi. Une molécule de type pi est composée d'un atome ou groupe d'atomes à travers lequel des électrons peuvent être délocalisés. De préférence les orbitales permettant cette délocalisation des électrons sont les orbitales p d'atomes de carbone à liaison multiple telles que par exemple celles des alcènes, alcynes, noyaux aromatiques neutres ou chargés, systèmes cycliques comprenant des hétéroatomes.

Le chromophore a une capacité à absorber l'énergie des photons dans la gamme du spectre visible, ainsi les électrons délocalisés pourront entrer en résonance avec le rayonnement incident. Ces molécules changeront donc de couleur en réponse à l'excitation lumineuse.

Le chromophore montre des propriétés optoélectroniques, ainsi il est utilisé pour la fabrication de composants électroniques qui émettent ou interagissent avec la lumière, il permet de véhiculer, mémoriser et d'amplifier des signaux optiques par des moyens purement optiques.

Récemment, les présents inventeurs ont rapporté une nouvelle classe de composés fortement fluorescents et électroluminescents en solution et à l'état solide en utilisant un fragment cyclohexyle comme agent de structure dirigeant l'agrégation du chromophore vers une agrégation de type J^{[1]}. La fonction imide introduite permet notamment d'accorder les propriétés électroniques vers un meilleur équilibre entre la mobilité des trous et celle des électrons mais également une fonctionnalisation aisée du chromophore. De la même manière, en utilisant la fonction triéthoxysilane en tant qu'agent structurant, les présents inventeurs ont pu synthétiser un précurseur hybride fortement fluorescent et électroluminescent ^{[2]}. Le matériau issu de l'hydrolyse-polycondensation classique du précurseur, montre une modification de l'organisation supramoléculaire du chromophore d'une agrégation de type J vers une agrégation de type H. Les films hybrides ainsi produits ne présentent plus de propriétés de fluorescence mais des photoconductivités élevées.

Il était donc nécessaire de mettre à disposition de l'homme du métier des composés chromophores qui présentent des propriétés de conductivités élevées tout en étant fortement fluorescents.

Les présents inventeurs ont trouvé que les composés de l'invention présentaient ces deux propriétés.

L'invention porte donc sur des composés chromophores de formule (I) ci-dessous : 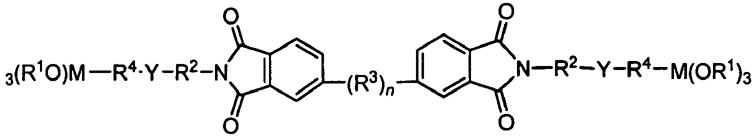 dans laquelle :
R¹ représente alkyle en C₁-C₄;
R² représente un groupement à encombrement stérique ;
R³ représente un système pi-conjugué ;
Y représente O, S, NH ou est absent;
R⁴ représente un groupe alkyle en C₃-C₁₈, de préférence en C₃-C₈, et plus préférentiellement encore en C₃;
M représente un métal du groupe III ou IV de la classification périodique ;
n est un nombre entier.

Le composé chromophore est ou bien un polymère ou bien un oligomère, selon la valeur du nombre entier n. Le nombre entier n peut être compris entre 1 et 10000, de préférence entre 1 et 100 et plus préférentiellement encore entre 1 et 20. Il est cependant plus aisé de travailler avec des oligomères ou de petits polymères pour lesquels n est compris entre 1 et 19, de préférence entre 1 et 15, et plus préférentiellement encore entre 1 et 10.

Par alkyle en C₁-C₄ on entend méthyle, éthyle, n-propyle, isopropyle, n-butyle, isobutyle, tert-butyle.

Comme métal M, on peut citer Si, Ti, Zr, Sn, Al. De préférence M est Si.

Comme exemple de groupement R² à encombrement stérique on peut citer les groupements choisis dans le groupe comprenant :

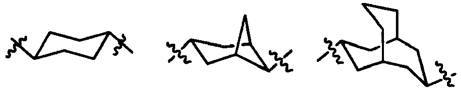

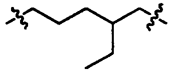

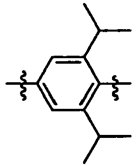

Comme exemple de système pi-conjugué R³, on peut citer ceux choisis dans le groupe comprenant : 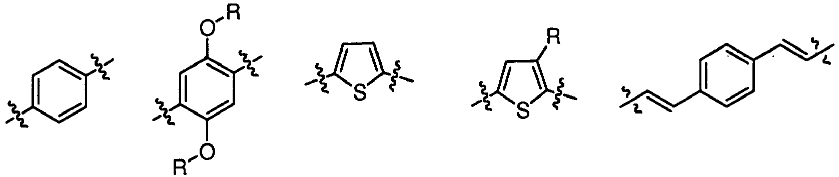 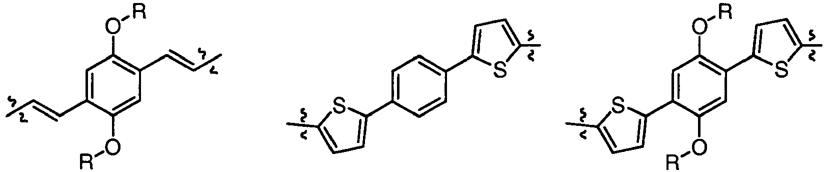 avec R représentant H ou un groupement alkyle en C₁-C₁₈, de préférence en C₁-C₁₂, et plus préférentiellement encore en C₁-C₈, le dit groupe alkyle étant éventuellement ramifié ou substitué par un hétéroatome (O, S, N...).

Ce nouveau chromophore, précurseur de matériaux hybrides fortement fluorescents, présente d'excellentes propriétés optoélectroniques. Le groupe M(OR¹)₃ réticulable permet la mise en oeuvre du procédé sol-gel. Le groupe à encombrement stérique évite l'agrégation après hydrolyse-polycondensation, ce qui permet de préserver les propriétés d'émission dans le matériau hybride.

Ce nouveau chromophore est préparé par un procédé qui comprend une étape consistant en l'introduction du groupement encombrant R² par condensation d'une amine aliphatique ou aromatique contenant ce groupement sur l'anhydride 4-bromo-phtalique. Cette étape du procédé sera décrite plus en détail ci-après.

Le procédé de préparation de chromophore conforme à l'invention comprend une étape d'hydrométallation d'un hydrogénotrialkoxy-métal [HM(OR¹)₃], catalysée par catalyseur de Karsted. L'hydrométallation est conduite sur le produit de condensation de l'anhydride-4-bromophtalique et de l'amine aromatique ou aliphatique.

Le procédé comprend en outre une étape ultérieure de couplage organométallique entre l'intermédiaire trialkoxymétallé et un système pi-conjugué comprenant R³.

Le mode d'introduction du groupement encombrant R² va dépendre de sa nature.

Dans le cas d'un groupement encombrant R² aliphatique, son introduction s'effectue selon le procédé général suivant : 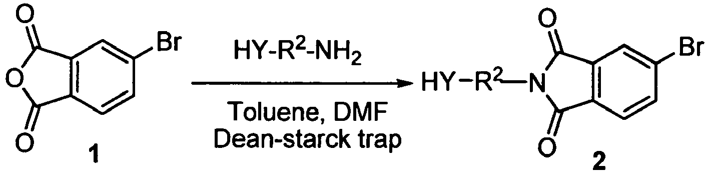 dans lequel R² et Y sont tels que définis précédemment.

Selon un mode de réalisation préféré, HYR²-NH₂ est un dérivé aminoalcool du groupement encombrant, de type HO-R²-NH₂.
Des exemples d'amino-alcools pouvant être utilisés sont :

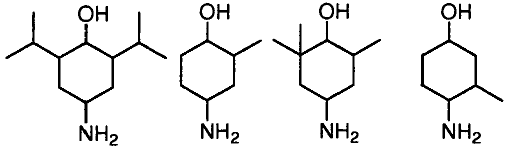

Sur le composé 2, modifié par insertion d'un groupement R'⁴, (R'⁴ représentant un groupe alkyle présentant le même nombre de carbone que le groupe R⁴ et présentant une insaturation éthylénique), on effectue la réaction d'hydrométallation selon le procédé général suivant : 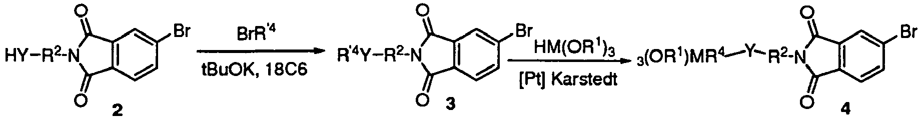 R¹, R², R³, R⁴ et Y étant tels que définis ci-dessus.

De façon particulière, le procédé est alors réalisé selon le schéma réactionnel suivant :

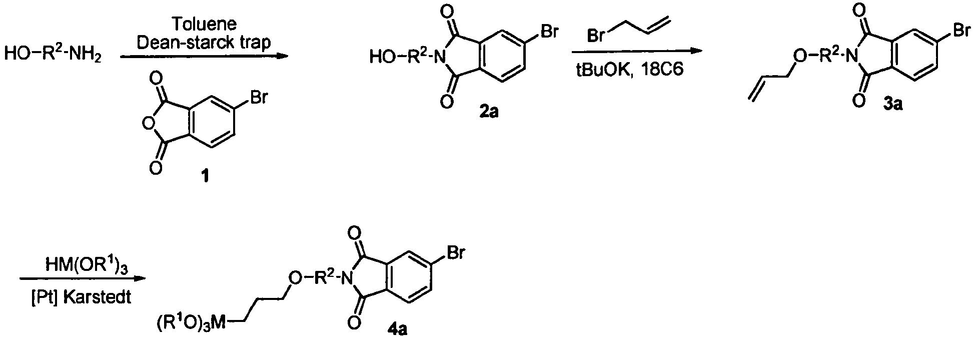

L'amino-alcool est tout d'abord condensé sur l'anhydride 1 au reflux dans le toluène dans un montage Deanstarck pour obtenir l'imido-alcool **2a.** L'alkylation de l'alcool par le bromure d'allyle en présence d'une base fourni **3a** avec de bons rendements. L'hydrosillylation de la fonction vinyle par HM(OR¹)₃ catalysée par le catalyseur de Karsted fournit l'intermédiaire **4a.**

Dans le cas d'un groupement encombrant R² aromatique, son introduction se fait selon le schéma réactionnel suivant :

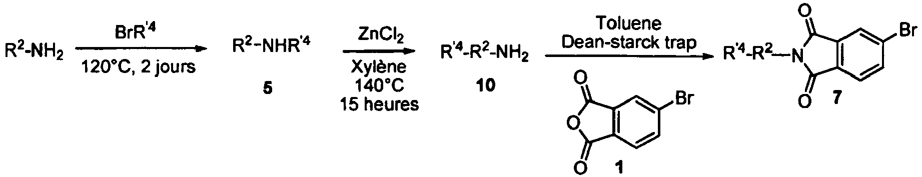

A titre d'exemple de R²-NH₂, on peut citer les benzamines suivantes :

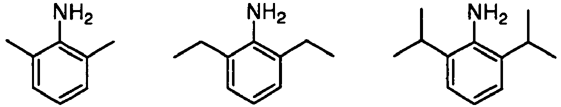

Sur le composé 7, on effectue la réaction d'hydrométallation selon le procédé général suivant :

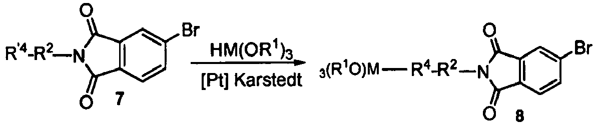

Selon un mode de réalisation préféré, ce procédé met en oeuvre une benzamine dérivée du groupement encombrant de type R²-NH₂, selon le schéma réactionnel suivant :

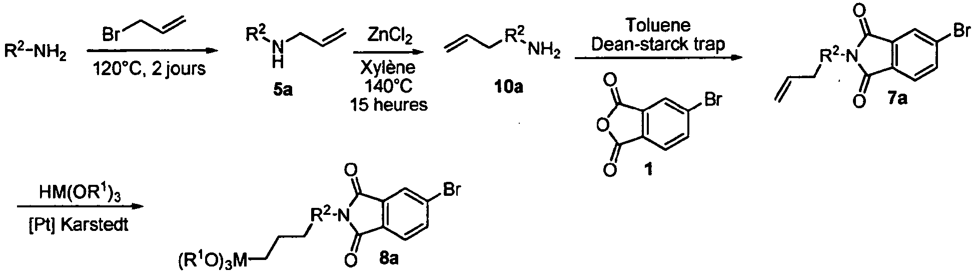

Dans un premier temps l'allylation de la fonction amine s'effectue par chauffage à 120°C pendant deux jours d'un mélange équimolaire de benzamine et de bromure d'allyle.

L'étape clé concerne le réarrangement de la N-allyl-benzamine **5a** en *para*-allyl-benzamine **10a.** Il s'effectue par action du chlorure de zinc au reflux du xylène avec d'excellent rendement (≈ 80%).

L'hydrosilylation de la fonction vinyle par HM(OR¹)₃ catalysée par le catalyseur de Karsted fournit l'intermédiaire **8a.**

Finalement, deux intermédiaires trialkoxymétallés ou bien de formule **4** (ou **4a**) ou bien de formule (**8** ou **8a**) sont engagés dans un couplage organométallique avec le système pi-conjugué.

Selon un mode préféré de l'invention, le chromophore présente la formule 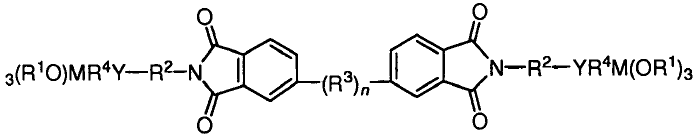 avec M=Si, R¹= éthyle, R²= cyclohexyle, n=1, R³=1,4-bis(octyloxy)-2,5-divinylbenzène, R⁴=-CH₂CH₂CH₂- et Y = O.

Ce composé est préparé selon le schéma réactionnel suivant :

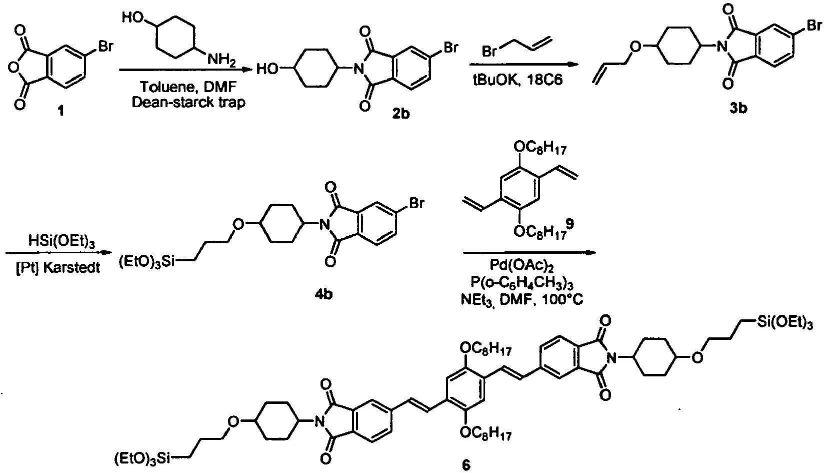

Dans un premier temps, le trans-aminocyclohexanole et l'anhydride 1^{[3]} sont condensés en chauffant au reflux dans le toluène dans un montage Dean-Stark pour fournir **2b** après recristallisation du produit brut. L'alkylation de l'alcool par le bromure d'allyle en présence d'une base a donné **3b** avec un bon rendement. L'hydrosilylation de la fonction vinyle par HSi(OEt)₃ catalysée par le catalyseur de Karsted fournit l'intermédiaire **4b** isolé par chromatographie sur gel de silice. **4b** est couplé au 1,4-divinyl-2,5-bis(octyloxy)benzène **9** en présence d'un catalyseur au palladium. La réaction de Heck est réalisée dans le N,N-diméthylformamide sec en présence de Pd(OAc)₂ et de P(*o*-C6H4Me)₃. Finalement, le précurseur conjugué **6** a été isolé par recristallisation dans l'éthanol pour fournir une poudre jaune avec un rendement de 24%. Ce nouveau précurseur silylé est soluble dans la plupart des solvants organiques, fortement fluorescents et émet dans le vert sous une irradiation à 430 nm dans le tétrahydrofurane (THF) [THF, ν_{abs_max} = 432 nm ; ν_{em_max} = 530 nm (νₑₓ = 430 nm)].

Le chromophore selon l'invention peut être utilisé dans un procédé sol-gel de formation de film mince. Il constitue un excellent précurseur de film mince qui est tout à la fois fluorescent et présente d'excellentes propriétés de photoconductivité.

Ainsi l'invention porte également sur l'utilisation d'un chromophore tel que défini ci-dessus ou tel que préparé ci-dessus, comme précurseur de film mince dans un procédé sol-gel.

Tout particulièrement, l'invention porte sur une méthode de stabilisation de l'agrégation de type J dans un film mince obtenu par procédé sol-gel, caractérisé par le fait que le chromophore précurseur du film mince comporte un groupement à encombrement stérique lié de façon covalente au groupement réticulale. De façon avantageuse, dans cette méthode le groupement à encombrement stérique est choisi dans le groupe comprenant 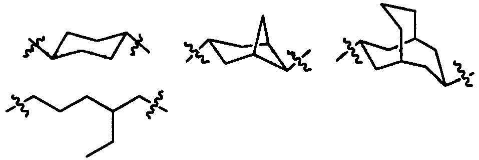 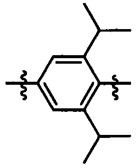 et le groupe réticulable est un groupe silylé. De préférence le groupe à encombrement stérique est le cyclohexyle et le groupe réticulable est le triméthoxysilyle ou le triéthoxysilyle.

La présente invention va être décrite plus en détails à l'aide des exemples suivants qui sont donnés à titre purement illustratif et qui ne sauraient en limiter la portée.

### EXEMPLES

### EXEMPLE 1 :

A une solution de 5-bromo-isobenzofuran-1,3-dione 1 (2g, 8,81mol) dans 150ML de toluène et 50 mL de DMF, on a ajouté du 4-aminocyclohexanol (1,04g, 8,81 mmol) et 1 mL de triéthylamine. Après reflux dans un montage Dean-Stark pendant une nuit à 140°C, le solvant a été évaporé et le résidu purifié par recristallisation dans l'éthanol. On a obtenu 1,7 g de composé de formule **2b** sous la forme d'un solide blanc (rendement : 58%).
p.f. :196-197 °C,
RMN⁻¹H (CDCl₃) δppm: 7.94 (d, *J =* 1.2 Hz, 1H); 7.83 (dd, *J* = 8, 1.6 Hz, 1H); 7.67 (d, *J =* 8 Hz, 1H); 4.11 (tt, *J =* 12.4, 4 Hz, 1H); 3.75 (tt, *J =* 11.2, 4.4 Hz, 1H); 2.31 (m, 2H); 2.09 (m, 2H); 1.74 (m, 2H); 1.43 (m, 2H).
RMN¹³C (CDCl₃) δppm: 167.5; 166.9; 136.8; 133.6; 130.4; 128.8; 126.5; 124.5; 69.5; 49.9; 34.7; 27.4.
IR max/cm⁻¹: 3326; 2935; 2862; 1769; 1709; 1606; 1459; 1417; 1374; 1170; 1152; 1092; 1063; 901; 740.
HR MS (FAB⁺; NBA): m/z = 324.0243 (M+H); calc. pour C₁₄H₁₄BrNO₃ : 324.0235.

A une solution du composé de formule **2b** ci-dessus (3,8g, 11,7 mmol) dans 50 mL de THF anhydre, on a ajouté sous atmosphère d'azote un éther couronne (30mg, 11,7 mmol), du tert-butylate de potassium (1,3 g, 11,7 mmol) puis, à 0°C, par fractions, du bromure d'allyle (7g, 57,8 mmol). Après agitation à température ambiante pendant 2 heures, la solution a été hydrolysée avec 40 mL d'eau. Le THF a été éliminé et le résidu a été extrait avec du dichlorométhane (3 x 50 mL). La couche organique a été séchée sur Na₂SO₄ et concentrée. Le produit a été purifié par chromatographie sur colonne (silice dichlorométhane) pour donner 2,35 g de 2-(4(allyloxy)cyclohexyl)-5-bromoisoindoline-1,3-dione de formule **3b** sous la forme d'un solide blanc (rendement 55%).
p.f. : 133-134°C
RMN ¹H (CDCl₃) δppm: 7.94 (d, *J* = 1.6 Hz, 1H) ; 7.83 (dd, *J* = 7.8, 2 Hz, 1H); 7.64 (d, *J* = 8 Hz, 1H); 5.93 (m, 1H); 5.28 (d, *J* = 17.2 Hz, 1H); 5.17 (d, *J* = 10.4 Hz, 1H); 4.12 (tt, *J* = 12.4, 4 Hz, 1H) ; 4.03 (d, *J* = 5.6 Hz; 2H); 3.40 (tt, *J* = 11.2, 4.4 Hz, 1H); 2.27 (m, 2H); 2.16 (m, 2H); 1.76 (m, 2H); 1.38 (m, 2H).
RMN ¹³C (CDCl₃) ppm: 167.6; 167.1; 137.0; 135.4; 133.7; 130.6; 128.9; 126.6; 124.6; 116.9; 76.2; 69.5; 50.3; 31.6; 27.7.
IR max/cm⁻¹: 2945; 2862; 1772; 1702; 1606; 1456; 1417; 1374; 1170; 1137; 1100; 1087; 1071; 920; 741.
HR MS (FAB⁺; NBA): m/z = 364.0539 (M+H); calc. pour C₁₇H₁₉BrNO₃: 364.0548.

A une solution du composé de formule **3b** (2g, 5,5 mmol), dans 10 ML de THF anhydre, à 0°C et sous atmosphère d'azote, on a ajouté du triéthoxysilane (1g, 6,08 mmol) et gouttes-à gouttes, du catalyseur Karsted (0,17 mL, 0,4%). Après 1 h à température ambiante, le THF a été éliminé et le résidu a été purifié par chromatographie flash (silice, dichlorométhane puis dichlorométhane/diéthyléther 2/1). On a obtenu 2,09g de 5-bromo-2-(4-(3-(triéthoxysilyl)-propyloxy)cyclohexyl)isoindoline-1,3-dione de formule **4b,** sous la forme d'un solide blanc (rendement 72%).
p.f. : 106°C.
RMN ¹H (CDCl₃) δppm: 7.94 (d, *J* = 1.6 Hz, 1H) ; 7.83 (dd, *J =* 8.2, 1.6 Hz, 1H) ; 7.67 (d, *J* = 8 Hz, 1H); 4.11 (tt, *J* = 12, 4 Hz, 1H); 3.83 (q, *J =* 6.8 Hz, 6H); 3.44 (d, *J =* 6.8 Hz, 2H); 3.33 (tt, *J =* 10.8, 4 Hz, 1H) ; 2.27 (m, 2H) ; 2.15 (m, 2H); 1.75 (m, 2H); 1.68 (m, 2H); 1.35 (m, 2H); 1.22 (t, *J* = 6.8 Hz, 9H); 0.64 (t, *J =* 8.4 Hz, 2H).
RMN ¹³C (CDCl₃) δppm: 167.6; 167.1; 137.0; 133.7; 130.6; 128.8; 126.6; 124.6; 76.7; 70.8; 58.5; 50.4; 31.7; 27.7; 23.5; 18.4; 6.6.
IR max/cm⁻¹: 2972; 2939; 2870; 1772; 1712; 1606; 1418; 1372; 1169; 1103; 1084; 958; 904; 742.
HR MS (FAB⁺; NBA): m/z = 528.1368 (M+H); calc. pour C₂₃H₃₅BrNO₆Si : 528.1359.

Un mélange du produit de formule **4b** ci-dessus (825 mg, 1,56 mmol), de 1,4-bis(octyloxy)-2,5-divinylbenzene (composé **9**) (300mg, 0,78 mmol), d'acétate de palladium (14,1 mg, 0,066 mmol) et de tri-o-tolylphosphine (76,8 mg, 0,26 mmol) dans 5 mL de THF anhydre a été chauffé , sous atmosphère d'azote à 100°C, puis on a ajouté de la triéthylamine (0,5 g, 0,17 mL, 5 mmol). Après 6h à 100°C et refroidissement, le solvant a été éliminé et le résidu a été purifié par recristallisation dans l'éthanol après une filtration à chaud destinée à l'élimination de l'insoluble. On a obtenu 260 mg de composé de formule 6 sous la forme d'un solide jaune (rendement : 24%).
p.f.: 181-182°C .
RMN ¹H (CDCl₃) δppm: 7.97 (s, 2H); 7.77 (m, 4H); 7.63 (d, J = 16.5 Hz, 2H); 7.26 (d, *J =* 16 Hz, 2H); 7. 12 (s, 2H); 4.14 (tt, *J* = 12.4, 4 Hz, 2H); 4.08 (t, *J* = 6.8 Hz, 4H); 3.83 (q, *J* = 7.2 Hz, 12H); 3.46 (t, *J* = 7.2 Hz, 4H); 3.56 (tt, *J* = 11.2, 4 Hz, 2H); 2.31 (m, 4H); 2.16 (m, 4H); 1.90 (qu, *J* = 7.2 Hz, 4H); 1.78 (m, 4H); 1.70 (m, 4H); 1.54 (qu, *J* = 8 Hz, 4H); 1.45 - 1.25 (m, 20H); 1.23 (t, *J* = 7.2 Hz, 18H); 0.87 (t, *J* = 6.8 Hz, 6H); 0.65 (t, *J* = 8.4 Hz, 4H).
RMN ¹³C (CDCl₃) δppm: 168.4; 168.2; 151.4; 151.4; 144.1; 132.9; 131.8; 130.1;127.6; 127.5; 126.7; 123.5; 120.4; 110.9; 70.7; 69.5; 58.4; 50.1; 31.8; 31.7; 29.4; 29.3; 27.7; 26.3; 23.5; 22.7; 18.3; 14.1; 6.6.
IR max/cm⁻¹: 2974; 2931; 2860; 1767; 1703; 1611; 1436; 1375; 1203; 1167; 1106; 1082; 961; 747.
Analyse élémentaire calc. pour C₇₂H₁₀₈N₂O₁₄Si₂: C 67.47, H 8.49, N 2.19; trouvé : C 66.57, H 8.26, N 2.11.

### EXEMPLE 2 :

On a préparé une solution de 30mg/mL de 5,5'-(1E,1'E)-2,2'-(2,5-bis(octyloxy)-1,4-phénylène)bis(éthène-2,1-diyl)bis(2-(4-(3-triéthoxysilyl)-propoxy)cyclohexyl)isoindoline-1,3-dione) (composé 6) dans le THF. A partir de cette solution, on a préparé un film mince de 100 nm par dépôt à la tournette (spin-coating en anglais) sur une plaque (wafer en anglais) de silicium.

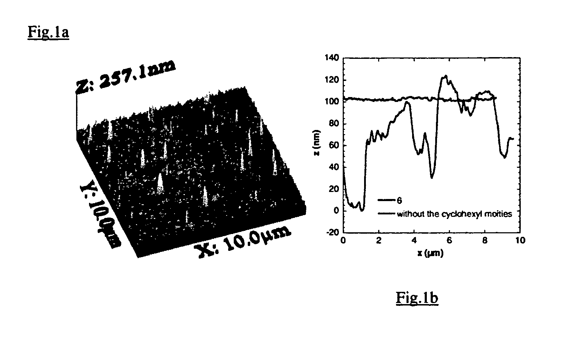

On a fait une étude topographique de couches déposées à la tournette sur plaque de silicium par microscopie de force atomique (AFM) en mode contact intermittent (tapping en anglais) à l'aide d'un appareil *Dimension 3100 Veeco Instruments.* L'image de la topographie de la plaque du composé 6 est donnée sur la figure la et le profil topographique est donné sur la figure 1b. Le profil topographique d'un film de 100 nm d'épaisseur à partir du précurseur ne possédant pas le groupement cyclohexyle ^{[2]} est également donné sur la figure 1b à titre de comparaison.

La rugosité du film obtenu avec le composé 6 en tant que précurseur est de 1 nm. Le film comparatif est quant à lui extrêmement hétérogène, sa rugosité est de 40 nm.
[1] G. Wantz, O. J. Dautel, R. Almairac, L. Hirsh, F. Serein-Spirau, L. Vignau, J.-P. Lere-Porte, J.P. Parneix, J. J. E. Moreau, Org. Elec., 2006, 7, 38.
[2] O. J. Dautel, G. Wantz, R. Almairac, D. Flot, L. Hirsh, J.-P. Lere-Porte, J.P. Parneix, F. Serein-Spirau, L. Vignau, J. J. E. Moreau, J. Am. Chem. Soc., 2006, 128, 4892.
[3] O. J. Dautel, G. Wantz, D. Flot, J.-P. Lere-Porte, J. J. E. Moreau, J.P. Parneix, F. Serein-Spirau, L. Vignau, J. Mat. Chem., 2005, 41, 4446.

## Revendications

1. Chromophore de formule générale 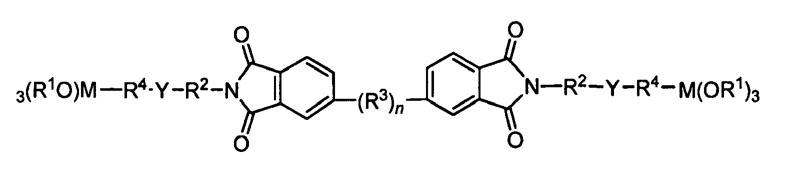 dans laquelle :
R¹ représente alkyle en C₁-C₄;
R² représente un groupement à encombrement stérique ;
R³ représente un système πpi-conjugué ;
Y représente O, S, NH ou est absent;
R⁴ représente un groupe alkyle en C₃-C₁₈, de préférence en C₃-C₈, et plus préférentiellement en C₃,
M représente un métal du groupe III ou IV de la classification périodique ;
n est un nombre entier.

2. Chromophore selon la revendication 1, **caractérisé par le fait que** M représente Si.

3. Chromophore selon la revendication 1 ou 2, **caractérisé par le fait que** le groupement à encombrement stérique est choisi dans le groupe comprenant :

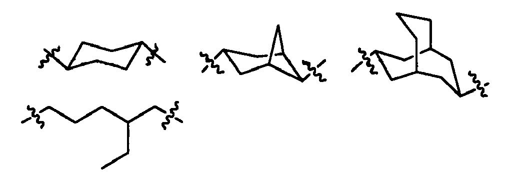

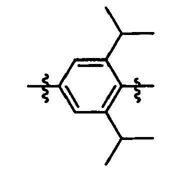

4. Chromophore selon l'une quelconque des revendications 1 à 3, **caractérisé par le fait que** R³ est choisi dans le groupe comprenant : 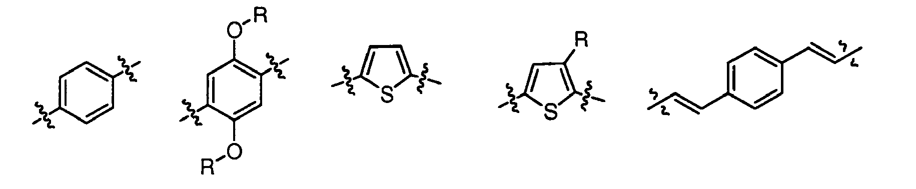 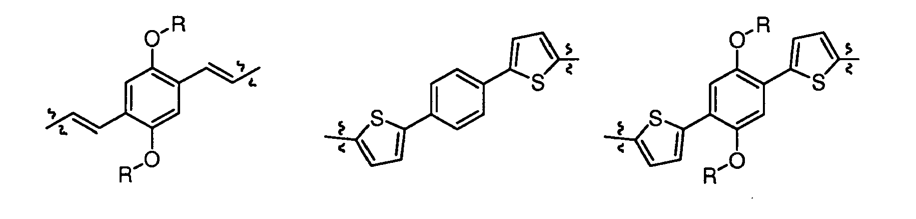 avec R représentant H ou un groupement alkyle en C₁-C₁₈, de préférence en C₁-C₁₂, et plus préférentiellement encore en C₁-C₆, ledit groupe alkyle étant éventuellement ramifié ou substitué par un hétéroatome (O, S, N...).

5. Chromophore selon l'une quelconque des revendications 1 à 4, qui est le 5,5'-(1E,1'E)-2,2'-(2,5-bis(octyloxy)-1,4-phénylène)bis(éthène-2,1-diyl)bis(2-(4-(3-triéthoxysilyl)-propoxy)cyclohexyl)isoindoline-1,3-dione).

6. Procédé de préparation de chromophore selon l'une quelconque des revendications 1 à 5, **caractérisé par le fait qu'**il comprend une étape consistant en l'introduction du groupement encombrant R² par condensation d'une amine aliphatique ou aromatique contenant le groupement R² sur l'anhydride 4-bromo-phtalique.

7. Procédé selon la revendication 6, qui est conduit selon l'un des schémas réactionnels suivants : 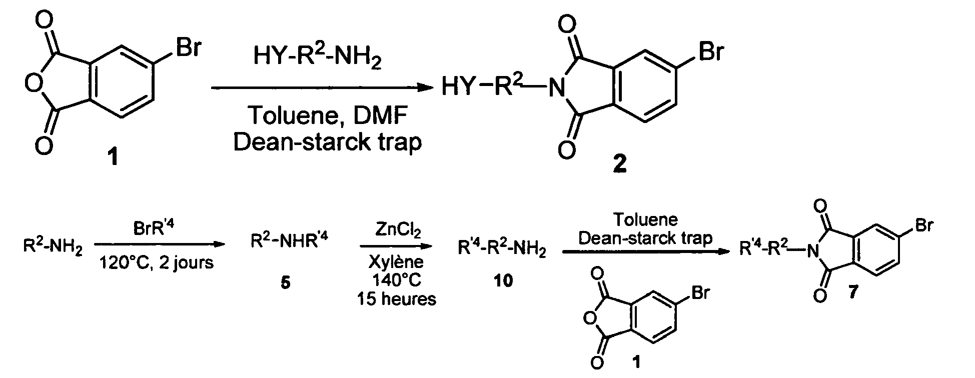 R² et Y étant tels que définis à la revendication 1 et R'⁴ représentant un groupe alkyle présentant le même nombre de carbone que le groupe R⁴ et présentant une insaturation éthylénique.

8. Procédé de préparation de chromophore selon l'une quelconque des revendications 1 à 5, **caractérisé par le fait qu'**il comprend une étape d'hydrométallation d'un hydrogénotrialkoxy-métal, catalysée par catalyseur de Karsted.

9. Procédé de préparation selon la revendication 8, qui est conduit selon l'un des schémas réactionnels suivants : 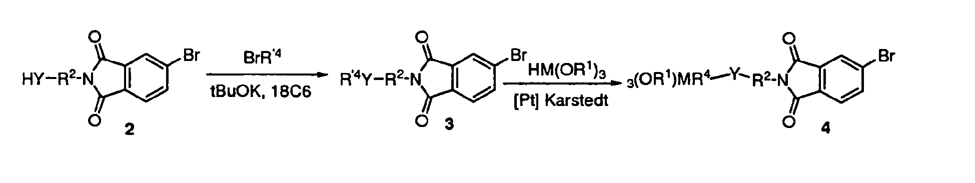 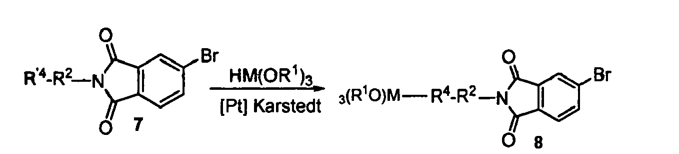 R¹, R², R⁴ et Y étant tels que définis à la revendication 1 et R'⁴ représentant un groupe alkyle présentant le même nombre de carbone que le groupe R⁴ et présentant une insaturation éthylénique.

10. Procédé selon la revendication 8 ou 9, qui comprend une étape ultérieure de couplage organométallique entre l'intermédiaire trialkoxymétallé et un système pi-conjugué comprenant R³, R³ étant tel que défini à la revendication 1.

11. Procédé de selon l'une quelconque des revendications 6 à 10, qui est conduit selon le schéma réactionnel suivant :

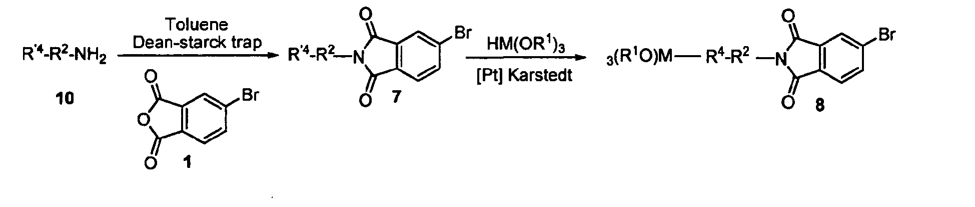

12. Procédé selon l'une quelconque des revendications 6 à 10, qui est conduit selon le schéma réactionnel suivant :

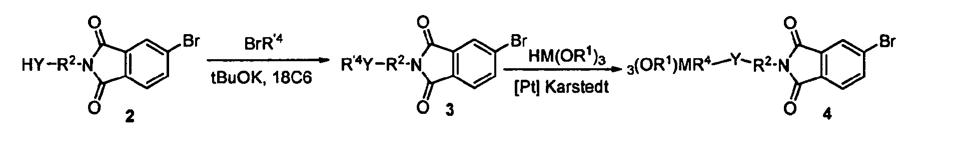

13. Procédé de préparation d'un chromophore selon la revendication 5, qui est mis en oeuvre selon le schéma réactionnel suivant :

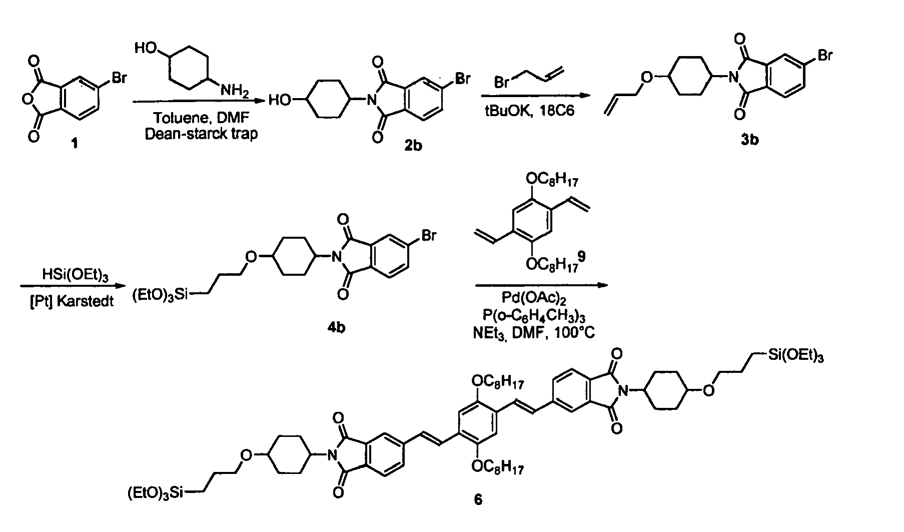

14. Utilisation d'un chromophore tel que défini à l'une quelconque des revendications 1 à 5, ou tel que préparé à l'une quelconque des revendications 6 à 13, comme précurseur de film mince dans un procédé sol-gel.

15. Méthode de stabilisation de l'agrégation de type J dans un film mince obtenu par procédé sol-gel, **caractérisé par le fait que** le chromophore précurseur du film mince comporte un groupement à encombrement stérique lié de façon covalente au groupement réticulale tel que défini à l'une des revendications 1 à 5.

16. Méthode selon la revendication 15, **caractérisée par le fait que** le groupement à encombrement stérique est choisi dans le groupe comprenant 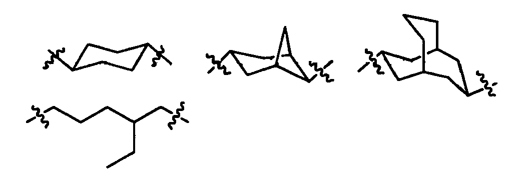 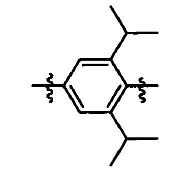 et le groupe réticulable est un groupe silylé.

17. Méthode selon la revendication 15 ou 16, **caractérisée par le fait que** le groupe à encombrement stérique est le cyclohexyle et le groupe réticulable est le triméthoxysilyle ou le triéthoxysilyle.

## Patentansprüche

1. Chromophor der allgemeinen Formel 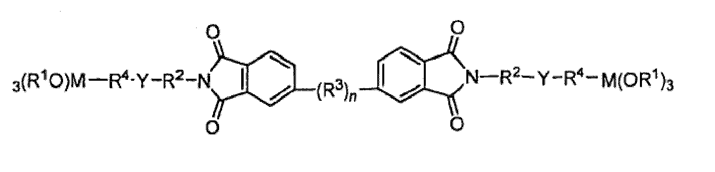 wobei:
R¹ eine C₁-C₄-Alkylgruppe darstellt;
R² eine sterisch gehinderte Gruppe darstellt;
R³ ein pi-konjugiertes System darstellt;
Y O, S, NH darstellt oder abwesend ist;
R⁴ eine C₃-C₁₈ Alkylgruppe, vorzugsweise eine C₃-C₈ Alkylgruppe, und
mehr bevorzugt eine C₃-Alkylgruppe darstellt;
M ein Metall der Gruppe III oder IV des Periodensystems der Elemente darstellt;
n eine ganze Zahl ist.

2. Chromophor nach Anspruch 1, **dadurch gekennzeichnet, dass** M Si darstellt.

3. Chromophor nach Anspruch 1 oder 2, **dadurch gekennzeichnet, dass** die sterisch gehinderte Gruppe ausgewählt ist aus der Gruppe umfassend:

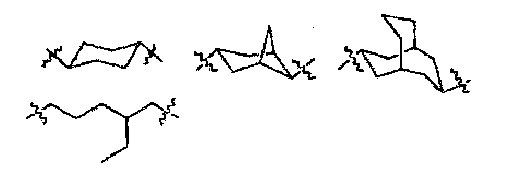

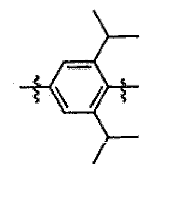

4. Chromophor nach einem der Ansprüche 1 - 3, **dadurch gekennzeichnet, dass** R³ ausgewählt ist aus der Gruppe umfassend: 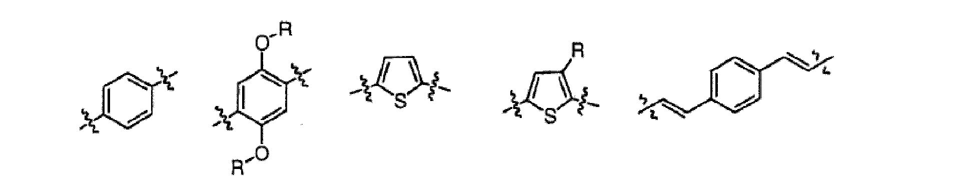 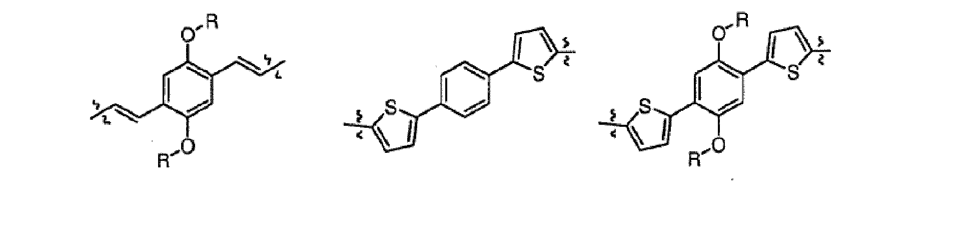 wobei R, H oder eine C₁-C₁₈ Alkylgruppe, vorzugsweise eine C₁-C₁₂ Alkylgruppe, mehr bevorzugt eine C₁-C₆-Alkylgruppe darstellt, wobei die Alkylgruppe gegebenenfalls verzweigt oder mit einem Heteroatom (O, S, N...) substituiert ist.

5. Chromophor nach einem der Ansprüche 1 - 4, welches 5,5'-(1 E,1'E)-2,2'-(2,5-bis(octyloxy)-1,4-phenylen)bis(ethen-2,1-d iyl)bis(2-(4-(3-triethoxysilyl)-propoxy)cyclohexyl)isoindolin-1,3-dion) ist.

6. Verfahren zur Herstellung des Chromophors nach einem der Ansprüche 1 - 5, **dadurch gekennzeichnet, dass** es einen Schritt umfasst, bestehend aus dem Einführen der gehinderten Gruppe R² durch Kondensation eines aliphatischen oder aromatischen Amins, welches die R² Gruppe enthält, mit 4-Brom-Phthalsäureanhydrid.

7. Verfahren nach Anspruch 6, welches nach einem der folgenden Reaktionsschemata durchgeführt wird: 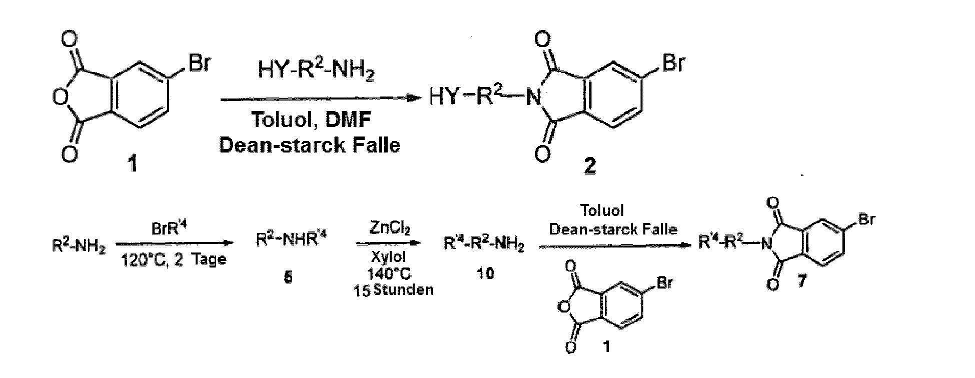 wobei R² und Y wie im Anspruch 1 definiert sind und R'⁴ eine Alkylgruppe darstellt, welche die gleiche Anzahl von Kohlenstoffatomen wie die Gruppe R⁴ und eine ethylenische ungesättigte Bindung aufweist.

8. Verfahren zur Herstellung eines Chromophors nach einem der Ansprüche 1 - 5, **dadurch gekennzeichnet, dass** es eine Hydrometallierung eines Hydrogentrialkoxymetalls umfasst, welche durch den Karsted-Katalysator katalysiert wird.

9. Verfahren zur Herstellung nach Anspruch 8, welches nach einem der folgenden Reaktionsschemata durchgeführt wird: 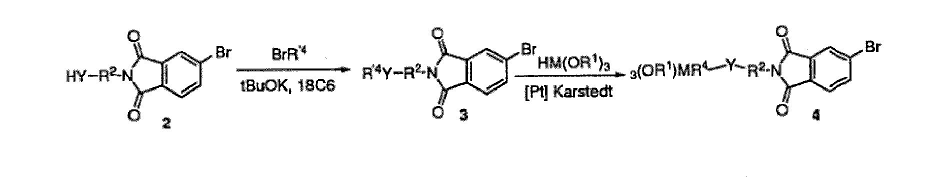 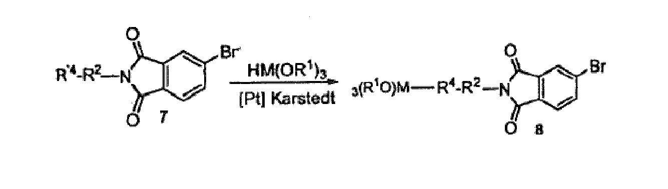 wobei R¹, R², R⁴ und Y wie im Anspruch 1 definiert sind und R'⁴ eine Alkylgruppe darstellt, welche die gleiche Anzahl von Kohlenstoffatomen wie die Gruppe R⁴ und eine ethylenisch ungesättigte Bindung aufweist.

10. Verfahren nach Anspruch 8 oder 9, welches einen nachfolgenden organometallischen Kupplungsschritt zwischen dem trialkoxymetallierten Zwischenprodukt und einem pi-konjugierten System, enthaltend R³, wobei R³ wie im Anspruch 1 definiert ist, umfasst.

11. Verfahren nach einem der Ansprüche 6 - 10, welches nach dem folgenden Reaktionsschema durchgeführt wird:

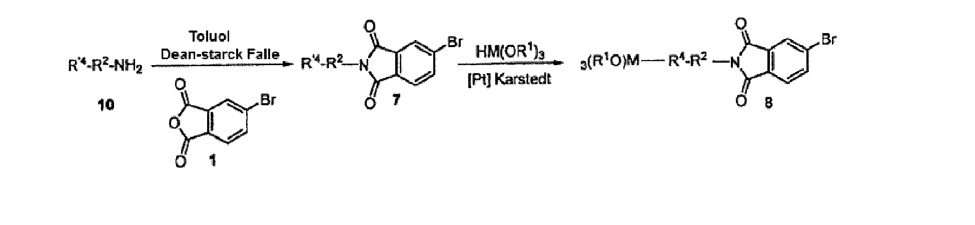

12. Verfahren nach einem der Ansprüche 6 - 10, welches nach dem folgenden Reaktionsschema durchgeführt wird:

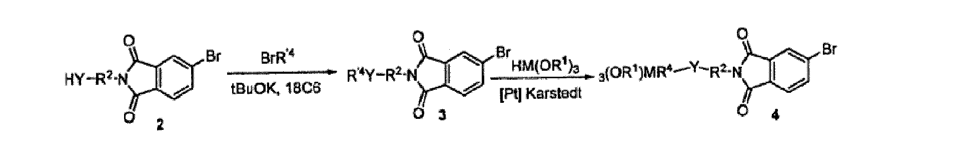

13. Verfahren zur Herstellung eines Chromophors nach Anspruch 5, welches nach dem folgenden Reaktionsschema durchgeführt wird:

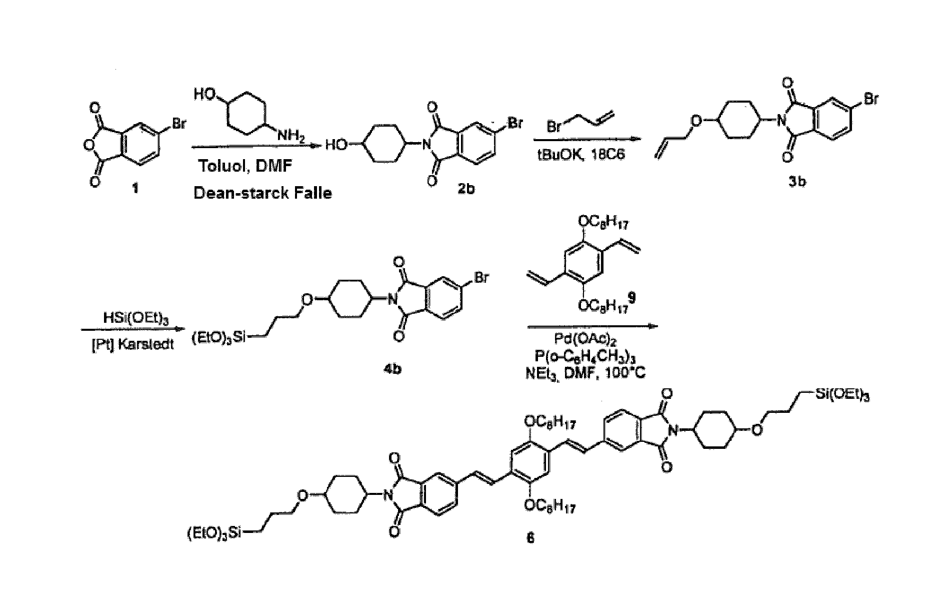

14. Verwendung eines Chromophors wie in einem der Ansprüche 1 - 5 definiert, oder wie nach einem der Ansprüche 6 - 13 hergestellt, als Vorstufe einer Dünnschicht in einem Sol-Gel-Verfahren.

15. Verfahren zur Stabilisierung der Aggregation vom J-Typ in einer Dünnschicht, welche durch ein Sol-Gel-Verfahren erhalten wurde, **dadurch gekennzeichnet, dass** ein Chromophor als Vorstufe der Dünnschicht eine sterisch gehinderte Gruppe enthält, welche kovalent an die vernetzbare Gruppe gebunden ist, wie in einem der Ansprüche 1 - 5 definiert.

16. Verfahren nach Anspruch 15, **dadurch gekennzeichnet, dass** die sterisch gehinderte Gruppe ausgewählt wird aus der Gruppe umfassend 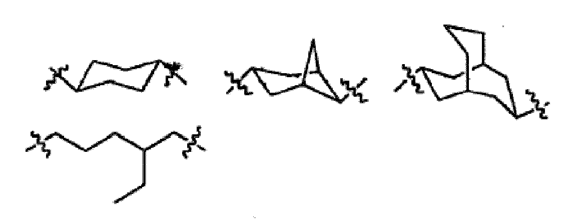 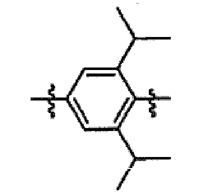 und die vernetzbare Gruppe eine Silylgruppe ist.

17. Verfahren nach Anspruch 15 oder 16, **dadurch gekennzeichnet, dass** die sterisch gehinderte Gruppe Cyclohexyl ist und die vernetzbare Gruppe Trimethoxysilyl oder Triethoxysilyl ist.

## Claims

1. A chromophore of general formula: 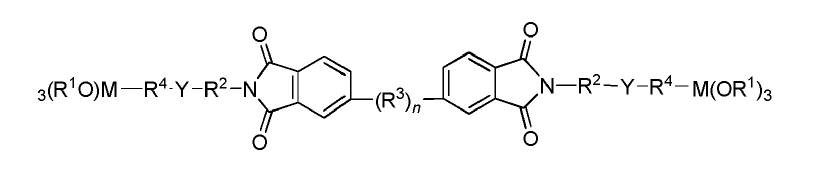 in which:
R¹ represents C₁-C₄ alkyl;
R² represents a sterically hindered group;
R³ represents a pi-conjugated system;
Y represents O, S, NH or is absent;
R⁴ represents a C₃-C₁₈, preferably C₃-C₈ and more preferably C₃ alkyl group;
M represents a metal from Group III or IV of the Periodic Table;
n is an integer.

2. The chromophore according to claim 1, **characterized in that** M represents Si.

3. The chromophore according to claim 1 or 2, **characterized in that** the sterically hindered group is chosen from the group comprising:

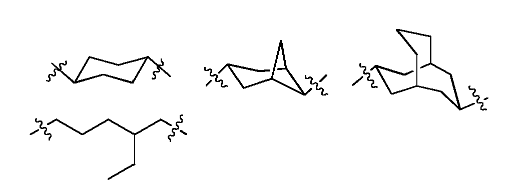

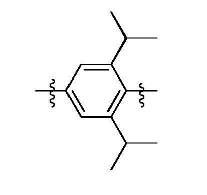

4. The chromophore according to any one of claims 1 to 3, **characterized in that** R³ is chosen from the group comprising: 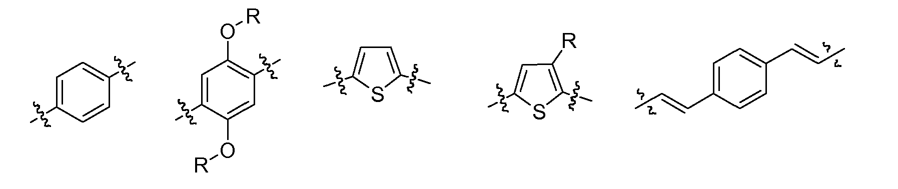 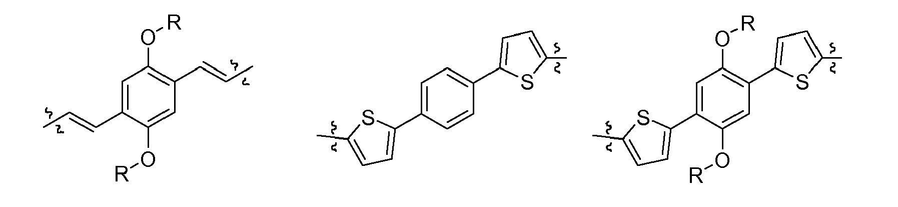 with R representing H or a C₁-C₁₈, preferably C₁-C₁₂ and more preferably still C₁-C₆ alkyl group, said alkyl group optionally being branched or substituted by a heteroatom (O, S, N...).

5. The chromophore according to any one of claims 1 to 4, which is 5,5'-(1E,1'E)-2,2'-(2,5-bis(octyloxy)-1,4-phenylene)bis(ethene-2,1-diyl)bis(2-(4-(3-(triethoxysilyl)-propoxy)cyclohexyl)isoindoline-1,3-dione).

6. A process for the preparation of a chromophore as defined in any one of claims 1 to 5, **characterized in that** it comprises a step consisting of the introduction of the bulky group R² by condensation of an aliphatic or aromatic amine comprising the R² group with 4-bromophthalic anhydride.

7. The process according to claim 6, which is carried out according to one of the following reaction schemes: 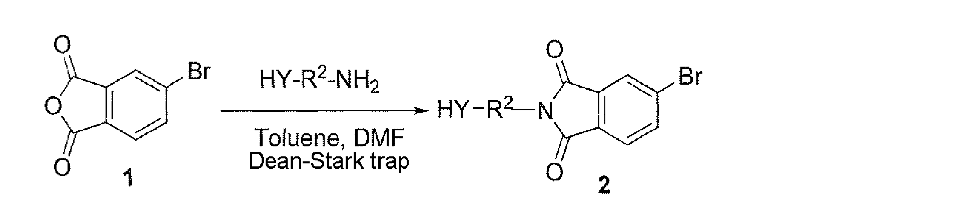 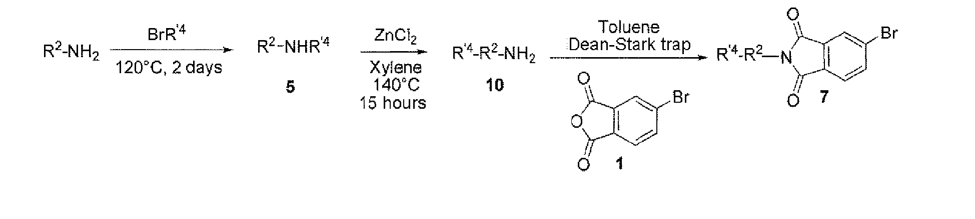 R² and Y being as defined in claim 1 and R'⁴ representing an alkyl group exhibiting the same carbon number as the R⁴ group and exhibiting an ethylenic unsaturation.

8. A process for the preparation of a chromophore as defined in any one of claims 1 to 5, **characterized in that** it comprises a step of hydrometallation of a hydrogenotrialkoxymetal catalyzed by Karstedt catalyst.

9. The preparation process according to claim 8, which is carried out according to one of the following reaction schemes: 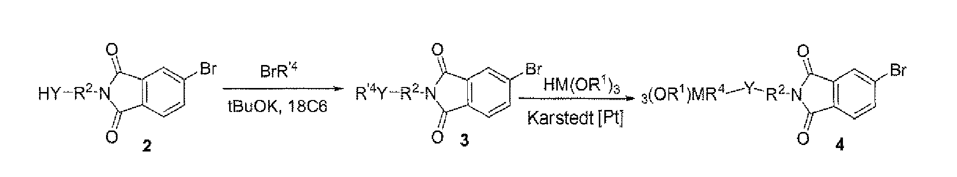 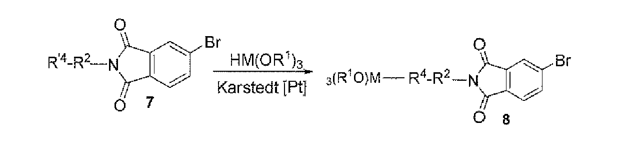 R¹, R², R⁴ and Y being as defined in claim 1 and R'⁴ representing an alkyl group exhibiting the same carbon number as the R⁴ group and exhibiting an ethylenic unsaturation.

10. The process according to claim 8 or 9, which comprises a subsequent step of organometallic coupling between the trialkoxymetallated intermediate and a pi-conjugated system comprising R³, R³ being as defined in claim 1.

11. The process according to any one of claims 6 to 10, which is carried out according to the following reaction scheme:

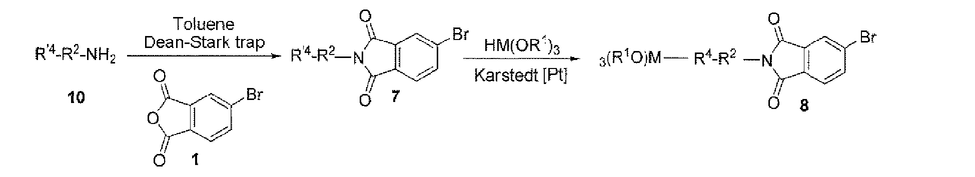

12. The process according to any one of claims 6 to 10, which is carried out according to the following reaction scheme:

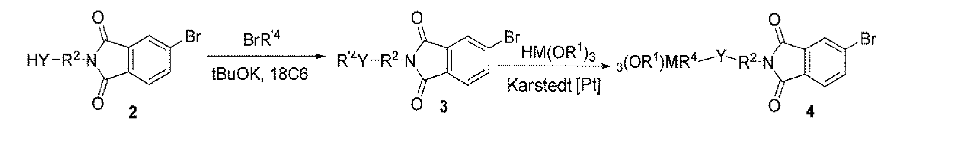

13. A process for the preparation of the chromophore as defined in claim 5, which is carried out according to the following reaction scheme:

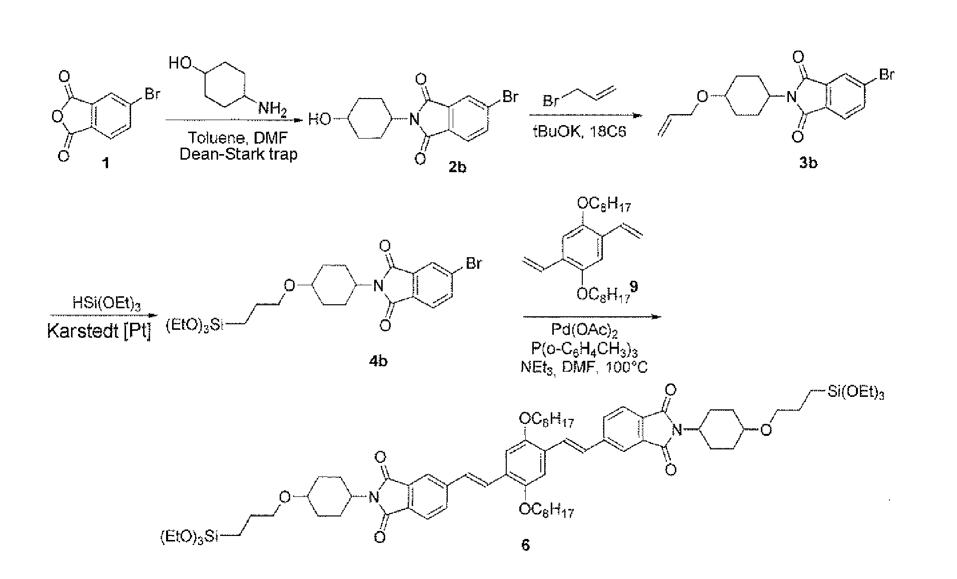

14. The use of a chromophore as defined in any one of claims 1 to 5 or as prepared in any one of claims 6 to 13 as thin film precursor in a sol-gel process.

15. A method for the stabilization of the J-type aggregation in a thin film obtained by a sol-gel process, **characterized in that** the precursor chromophore of the thin film comprises a sterically hindered group covalently bonded to the crosslinkable group as defined in one of claims 1 to 5.

16. The method according to claim 15, **characterized in that** the sterically hindered group is chosen from the group comprising: 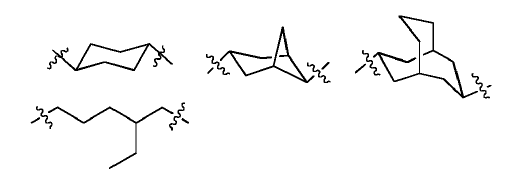 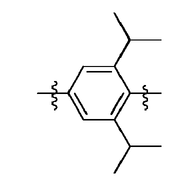 and the crosslinkable group is a silylated group.

17. The method according to claim 15 or 16, **characterized in that** the sterically hindered group is the cyclohexyl and the crosslinkable group is the trimethoxysilyl or the triethoxysilyl.
